# EUROPÄISCHE PATENTANMELDUNG

(11) **EP 1 627 729 A1**
(43) Veröffentlichungstag der Anmeldung: **22.02.2006**
(21) Anmeldenummer: 05107384.9
(22) Anmeldetag: 11.08.2005
(51) Int. Cl.: B31B 23/00, B65B 9/02, B65D 33/18, A61F 15/00

(54) **Verfahren zur Herstellung eines geschlossenen Verpackungsbeutels**

(30) Priorität: 21.08.2004 DE 102004040624
(71) Anmelder: HDG Verpackungsmaschinen GmbH, 51515 Kürten (DE)
(72) Erfinder: Wagner, Dirk, 51688 Wipperfürth-Thier (DE)
(74) Vertreter: Selting, Günther

(57) **Zusammenfassung**

Der Verpackungsbeutel (40) wird aus einer Materialbahn hergestellt, bei der ein abgeschnittener Streifen (14) mit dem verbliebenen Teil der Materialbahn durch eine Verbindungsnaht (30) verbunden wird. Durch eine längslaufende Falte (31) werden zwei Beutelwände gebildet, wobei der Beutel zunächst an seiner Unterseite offen ist und erst nach dem Befüllen durch eine Verbindungsnaht (13) geschlossen wird. Der Beutel kann entlang der ersten Verbindungsnaht (30) durch Ziehen an einer Lasche (44) geöffnet werden, ohne dass ein sich vom Beutel lösender Abfall entsteht. Der Beutel kann auch zur Wiederaufnahme des benutzten Gegenstandes (42) verwendet und anschließend wieder geschlossen werden.

## Beschreibung

Die Erfindung betrifft ein Verfahren zur Herstellung eines geschlossenen Verpackungsbeutels für einen Gegenstand.

Es ist bekannt, Verpackungsbeutel in Form von Flachbeuteln aus übereinander liegenden Folien herzustellen, die mit längslaufenden und querlaufenden Verbindungsnähten abgeschweißt werden. Solche Verpackungsbeutel können aus Aluminiumverbundfolie bestehen, die einseitig oder beidseitig mit Polyethylen beschichtet ist, um sie siegelfähig zu machen.

Die bekannten Verpackungsbeutel sind schwierig zu öffnen. In der Regel erfordern sie beim Öffnen das Abreißen einer Ecke oder eines Streifens. Dies ist nicht nur schwierig und kann einen erheblichen momentanen Kraftaufwand erfordern, sondern es führt auch dazu, dass mehrteiliger Abfall entsteht. Außerdem kann bei einem derartigen Öffnungsvorgang der Verpackungsbeutel unbrauchbar für eine Wiederaufnahme des benutzten Gegenstandes werden, weil er sich nicht hinreichend schließen lässt.

Ein Verfahren zur Herstellung eines geschlossenen Verpackungsbeutels, das im wesentlichen dem Oberbegriff des Patentanspruchs 1 entspricht, ist beschrieben in GB 1 232 248. Bei diesem Verfahren wird auf eine Materialbahn ein Streifen aufgebracht, so dass die Materialbahn eine einlagige Wand und eine Verbundwand erhält. Diese beiden Wände werden längs einer Falte übereinander gefaltet, wobei der zu verpackende Gegenstand zwischen den Wänden eingeschlossen wird. Dann werden die beiden Wände längs dreier Kanten durch Verbindungsnähte verbunden. In der Verbundwand ist ein Streifen auf der Außenseite der Materialbahn aufgeklebt. Dieser Klebebereich kann zur Entnahme des umschlossenen Gegenstandes geöffnet und ggf. wieder verschlossen werden. Der aufgesetzte Streifen der Verbundwand bildet eine Klappe zum Öffnen des Beutels.

In US 4,205,504 ist ein Verfahren zur Herstellung eines geschlossenen Verpackungsbeutels aus einer durchgehenden Materialbahn beschrieben. Hierbei wird die Materialbahn zunächst an einer Querstation entlang geführt, die Quernähte erzeugt. Dann wird die Materialbahn aufgeschnitten, wobei ein Teil der Materialbahn in Transportrichtung weiterläuft, während ein anderer Teil umgelenkt und quer über den ersten Teil der Materialbahn gelegt wird.

Der Erfindung liegt die Aufgabe zugrunde, ein Verfahren zur Herstellung eines geschlossenen Verpackungsbeutels anzugeben, das eine schnelle und einfache Herstellung ermöglicht, wobei der nach dem Verfahren hergestellte Beutel leicht zu ergreifen und zu öffnen ist.

Das erfindungsgemäße Verfahren ist durch den Patentanspruch 1 bezeichnet.

Das erfindungsgemäße Verfahren sieht folgende Schritte vor:
a) Aufbringen eines Streifens auf eine Materialbahn derart, dass beide eine Überlappungszone bilden,
b) Herstellung einer längslaufenden ersten Verbindungsnaht im Bereich der Überlappungszone,
c) Falten der Materialbahn um eine längslaufende Falte, wobei eine einlagige Wand und eine Verbundwand aus einem Abschnitt der Materialbahn und dem Streifen gebildet werden,
d) Verbinden der beiden übereinander liegenden Wände durch Quernähte,
e) Einfüllen des Gegenstandes in eine der Falte entgegengesetzte Öffnung,
f) Verschließen der Öffnung durch eine zweite Verbindungsnaht.

Das Verfahren ist dadurch gekennzeichnet, dass der Streifen sich an der Innenseite des Abschnitts befindet, und dass der als Träger für den Streifen dienende Abschnitt eine Klappe des Beutels bildet.

Der Verpackungsbeutel wird aus zwei Wänden gebildet, von denen die eine als Verbundwand bezeichnet werden kann. Die Verbundwand ist mit der einlagigen Wand einstückig verbunden und sie besteht aus dem Material der Materialbahn und dem damit verbundenen Streifen, wobei eine Überlappung gebildet wird. Die Überlappung bildet eine Lasche, an der der Beutel zum Aufreißen ergriffen werden kann, um längs der ersten Verbindungsnaht und der angrenzenden Quernähte aufgerissen zu werden.

Die nach dem erfindungsgemäßen Verfahren hergestellten Verpackungsbeutel eignen sich insbesondere zum Aufnehmen flacher Gegenstände, wie beispielsweise Putztücher oder Feuchttücher. Sie können aber auch für Produkte wie Trockenobst, Erdnüsse, Kleineisenwaren, Tabak benutzt werden. Sie bestehen aus feuchtigkeitsundurchlässigem Material, insbesondere aus Aluminiumverbundfolie, wobei auf einer Seite eine siegelfähige Kunststoffschicht und auf der anderen Seite eine Farbschicht angebracht sein kann. Die Kunststoffschicht muss nicht völlig kontinuierlich sein. Sie kann aus regelmäßigen oder unregelmäßigen Strukturen bestehen, wodurch eine bedingte Siegelfähigkeit erhalten wird, mit der Möglichkeit, die Siegelnaht manuell auseinander zu ziehen. Der hier verwendete Begriff "Gegenstand" umfasst jegliche Art von verpackungsfähigen Produkten, einschließlich solcher, die pulverförmig, granulatförmig, stückförmig (ein- oder mehrteilig) sind.

Mit dem erfindungsgemäßen Verfahren entsteht ein allseitig geschlossener Beutel, der entlang der Nähte zu öffnen ist, ohne Teile des Beutels abzureißen oder von dem Beutel zu entfernen.

Das Verfahren zur Herstellung eines Verpacktangsbeutels kann in der Weise durchgeführt werden, dass der Verpackungsbeutel aus einer einzigen Materialbahn gefertigt wird. Hierbei ist vorgesehen, dass das Merkmal a) in folgenden Unterschritten durchgeführt wird:
a1) Bntlangbewegen einer Materialbahn an einer Schneidvorrichtung zum Abtrennen des Streifens von der Materialbahn,
a2) Versetzen des Streifens relativ zu der Materialbahn seitlich zur Bewegungsrichtung zur Bildung der Überlappungszone zwischen dem Streifen und der Materialbahn.

Mit dem erfindungsgemäßen Verfahren kann ein Verpackungsbeutel hergestellt werden, der an einer der beiden Beutelwände eine Lasche aufweist, welche längs einer aufreißbaren Verbindungsnaht mit der Beutelwand verbunden ist. Die Lasche kann als Öffnungshilfe benutzt werden.

Eine Besonderheit des erfindungsgemäßen Verfahrens besteht darin, dass zunächst der obere Bereich des Beutels geformt wird, während der Beutel an der Seite, die später die Unterkante bildet, zunächst offen bleibt. Die Befüllung des Beutels mit dem Gegenstand erfolgt von der offenen Unterseite aus. Nach dem Befüllen wird der Beutel durch Herstellen der zweiten Verbindungsnaht geschlossen.

Im Folgenden wird ein Ausführungsbeispiel der Erfindung unter Bezugnahme auf die Zeichnungen näher erläutert.

### Es zeigen:

- Figur 1: eine Draufsicht auf eine Vorrichtung, die von einer Materialbahn während des Transportes über Rollen einen Streifen abtrennt und diesen Streifen überlappend auf die Materialbahn legt,
- Figur 2: eine Seitenansicht der Vorrichtung nach Figur 1,
- Figur 3: einen Querschnitt entlang der Linie III/III von Figur 1,
- Figur 4: einen Querschnitt entlang der Linie IV/IV von Figur 1,
- Figur 5: die Herstellung der längslaufenden ersten Verbindungsnaht zum Verbinden des Streifens mit der Materialbahn,
- Figur 6: den Vorgang des Faltens der Materialbahn,
- Figur 7: das Erzeugen von Quernähten und gleichzeitiges Abschneiden der Verpackungsbeutel,
- Figur 8: das Öffnen des Beutels an der noch offenen Beutelkante,
- Figur 9: das Einführen des Gegenstandes in den Beutel,
- Figur 10: das Schließen des Beutels durch Herstellen der zweiten Verbindungsnaht,
- Figur 11: eine Darstellung des Beutels in der Gebrauchslage und des Öffnens des Beutels durch Ziehen an der Lasche, und
- Figur 12: eine Darstellung des geöffneten Beutels und der Entnahme des Gegenstandes aus dem Beutel.

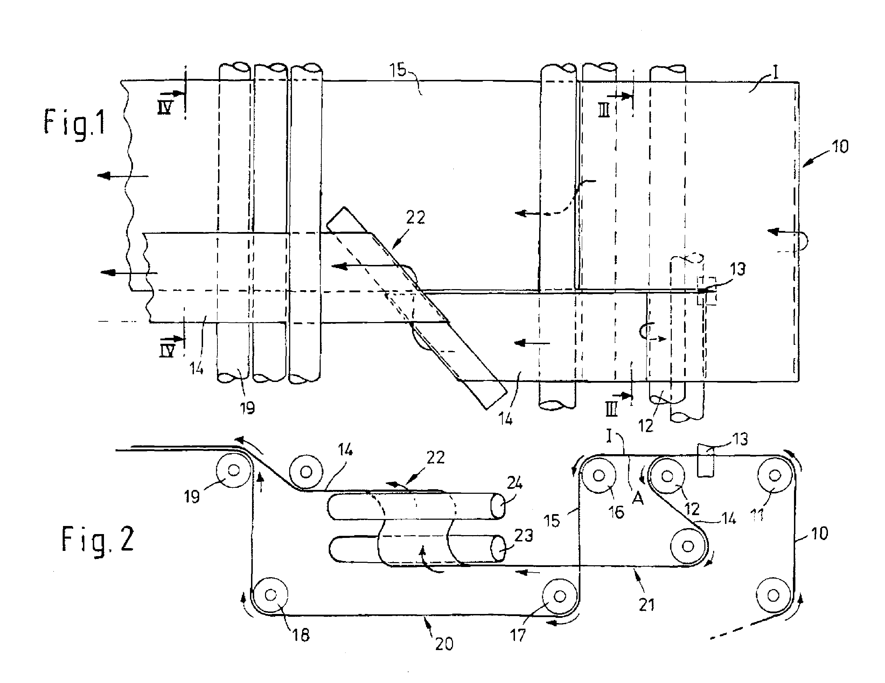

In den Figuren 1 und 2 ist eine Materialbahn 10 dargestellt, aus der der Verpackungsbeutel hergestellt wird. Die Materialbahn 10 ist bei dem hier beschriebenen Beispiel eine Aluminiumfolie, die eine Innenseite I und eine Außenseite A hat. Die Innenseite und die Außenseite bezeichnen die Orientierung dieser Seiten bei dem herzustellenden Beutel. Die Innenseite I trägt eine Beschichtung aus PE, die einerseits eine Schutzschicht für die Oberfläche des Aluminiums und andererseits eine siegelfähige Schicht bildet. Die Außenseite A ist bei dem vorliegenden Ausführungsbeispiel mit einer Bedruckung versehen. Sie kann zusätzlich mit einer PE-Schicht bedeckt sein.

Die Materialbahn 10 läuft über Rollen 11, 12, zwischen denen eine Schneidvorrichtung 13 in Form einer Klinge angeordnet ist. Die Schneidvorrichtung 13 trennt von der Materialbahn 10 einen Streifen 14 ab. Der verbleibende Teil der Materialbahn 10, der um die Breite des Streifens 14 verringert ist, ist mit 15 bezeichnet. Dieser Teil 15 der Materialbahn läuft über Rollen 16, 17, 18, 19 in der ursprünglichen Richtung weiter, wobei eine nach unten gerichtete Schleife 20 gebildet wird. Der Streifen 14 wird von der Rolle 12 aus dem Weg des Teils 15 der Materialbahn abgelenkt und durchläuft eine Schleife 21, die zunächst seitlich neben dem verbleibenden Teil 15 der Materialbahn verläuft. Im Zuge der Schleife 21 ist eine Versatzvorrichtung 22 angeordnet, die eine seitliche Verschiebung des Streifens 14 in Bezug auf den Teil 15 vornimmt. Die Versatzvorrichtung 22 weist parallele Umlenkrollen 23, 24 auf, deren Achsen schräg zur Bewegungsrichtung der Materialbahn verlaufen. Der Streifen 14 läuft s-förmig zunächst um die eine Umlenkrolle 23 und dann um die andere Umlenkrolle 24, wobei er über der Rolle 19 auf die Oberseite des Teils 15 der Materialbahn aufgelegt wird und diese teilweise Überlappt.

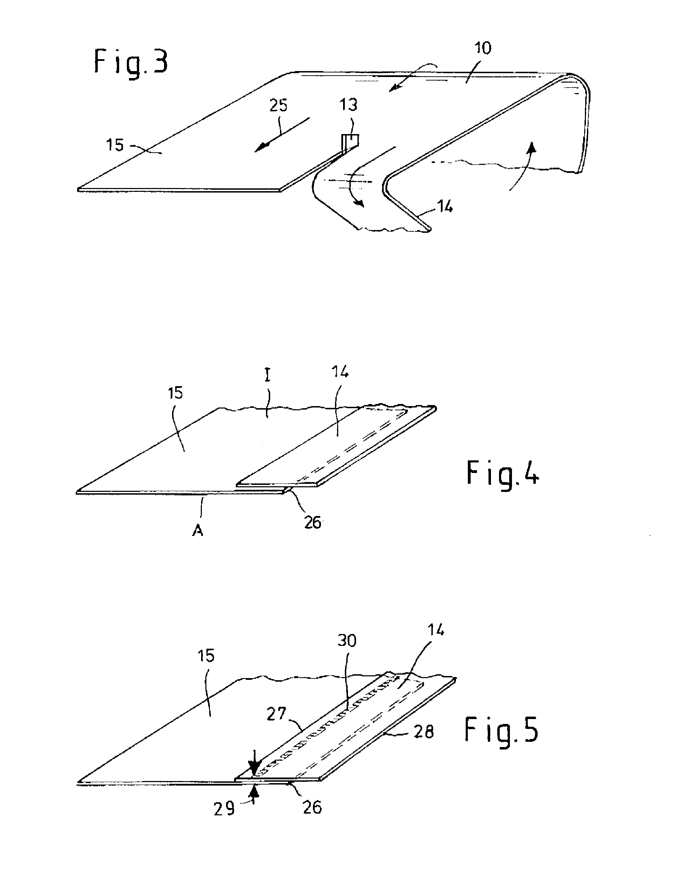

In Figur 3 ist der Vorgang des Abtrennens des Streifens 14 von der Materialbahn 10 unter Verwendung der Schneidvorrichtung 13 schematisch dargestellt. Die Bewegungsrichtung der Materialbahn ist mit 25 bezeichnet.

Figur 4 zeigt den Streifen 14, der den Materialbahnteil 15 teilweise überlappt. Der Streifen 14 steht über den Rand 26 des Materialbahnteils 15 seitlich über, wobei sein innerer Rand 27 sich über dem Materialbahnteil 15 befindet und der äußere Rand 28 außerhalb des Materialbahnteils.

Gemäß Figur 5 wird die längslaufende erste Verbindungsnaht 30 mit einer (nicht dargestellten) Heißsiegelungsvorrichtung erzeugt, an der die Folienkombination schrittweise entlanggeführt wird. Die Heißsiegelungsvorrichtung weist Siegelbacken auf, die von entgegengesetzten Seiten her gegen die Folienkombination gedrückt werden, was durch die Pfeile 29 angedeutet ist.

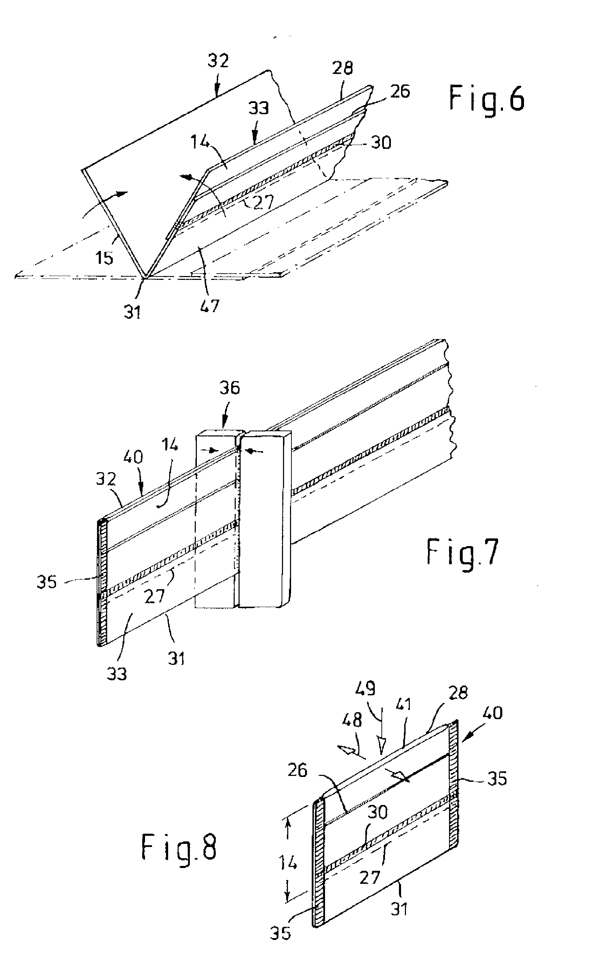

Gemäß Figur 6 wird die Folienkombination aus dem Materialbahnteil 15 und dem daran befestigten Streifen 14 entlang einer Faltvorrichtung transportiert, die eine längslaufende Falte 31 erzeugt. Dadurch werden zwei Wände des Beutels gebildet, nämlich die einlagige Wand 32 und die Verbundwand 33, die kongruent aufeinander passen. Der Streifen 14 befindet sich nun an der Innenseite der Verbundwand 33.

Figur 7 zeigt das Herstellen der Quernähte 35 durch eine Siegelungsvorrichtung 36, die zwei heiße Siegelungsbacken aufweist, welche von entgegengesetzten Seiten her gegen den Beutel gedrückt werden. Die Siegelungsbacken sind mit einer Trennvorrichtung kombiniert, die bei jedem Siegelungsvorgang einen Beutel 40 abtrennt.

In der Figur 8 ist der Beutel 40 dargestellt, wobei zwei entgegengesetzte Beutelenden durch Quernähte 35 verschlossen sind und ein längslaufender Rand durch die Falte 31 verschlossen ist. Der Falte 31 gegenüber befindet sich eine Öffnung 41, an derjenigen Kante, die später beim Gebrauch des Beutels die Unterkante bildet. Der Beutel wird an der Öffnung 41 durch Saugeinrichtungen entlang der Pfeile 48 auseinandergezogen und gleichzeitig wird Druckluft 49 in die Öffnung 41 geblasen.

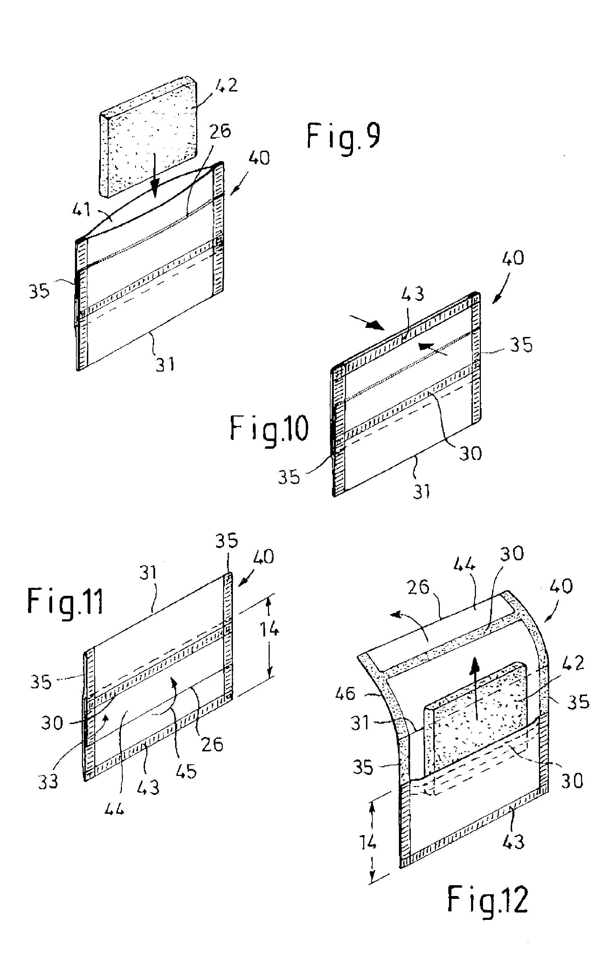

In Figur 9 ist das Einfüllen des Gegenstandes in die Öffnung 41 dargestellt. Anschließend wird gemäß Figur 10 die zweite Verbindungsnaht 43 als Siegelnaht hergestellt und somit der Beutel abdichtend geschlossen.

In Figur 11 ist der Beutel im gebrauchsfertigen Zustand dargestellt. Die Verbindungsnaht 43 bildet nunmehr die Unterkante und die Falte 31 die Oberkante des Flachbeutels. Die Verbindungsnaht 30 verläuft etwa auf mittlerer Höhe des Beutels. Sie ist unter der Verbundwand 33 verdeckt.

Die Rand 26 befindet sich im Abstand von der Verbindungsnaht 30, so dass eine Lasche 44 gebildet wird, die an den Enden von den Quernähten 35 begrenzt ist und oben von der Verbindungsnaht 30. Die Kante der Lasche 44 ist freiliegend. Die Lasche kann somit durch Untergreifen in Richtung des Pfeiles 45 hochgezogen werden, wodurch die Packung geöffnet wird.

Figur 12 zeigt den Beutel 40 in geöffnetem Zustand. Durch Ziehen an der Lasche 44 ist zunächst die erste Verbindungsnaht 30 aufgegangen und dann haben sich auch die Quernähte 35 bis hin zu Falte 31 fortlaufend gelöst. Man erkennt, dass der Gegenstand 42 nunmehr entnommen werden kann. Der Gegenstand kann nach seiner Benutzung im verschmutzten Zustand wieder in den Verpackungsbeutel eingelegt werden und anschließend kann die Klappe 46 verschlossen werden, wobei die beiden Hälften der Verbindungsnaht 30 gegeneinander gedrückt werden und jedenfalls annähernd aneinander haften.

Zur Erleichterung des Öffnens kann die Verbindungsnaht 30, die hier in Längsrichtung des Beutels verläuft auch schräg zur Längsrichtung verlaufen oder keilförmig ausgebildet sein, so dass der Lösungsvorgang beim Öffnen an einer definierten Stelle beginnt und sich anschließend fortsetzt. Der Abschnitt 47 des Materialbahnteils 15, der gemäß Figur 6 als Träger für den Streifen 14 dient, bildet gemäß Figur 12 die Klappe 46 des Beutels 40.

Die Erfindung ist nicht auf das oben beschriebene Ausführungsbeispiel beschränkt. Im Rahmen der Erfindung sind zahlreiche abgewandelte Ausführungsformen möglich. Der Schutzbereich wird durch die Patentansprüche bestimmt.

## Patentansprüche

1. Verfahren zur Herstellung eines geschlossenen Verpackungsbeutels (40) für einen Gegenstand (42), mit den Schritten
a) Aufbringen eines Streifens (14) auf eine Materialbahn (15) derart, dass beide eine Überlappungszone bilden,
b) Herstellen einer längslaufenden ersten Verbindungsnaht (30) im Bereich der Überlappung,
c) Falten der Materialbahn um eine längslaufende Falte (31), wobei eine einlagige Wand (32) und eine Verbundwand (33) aus einem Abschnitt (47) der Materialbahn (15) und dem Streifen (14) gebildet werden,
d) Verbinden der beiden übereinander gelegten Wände (32, 33) durch Quernähte (35),
e) Einfüllen des Gegenstandes (42) in eine der Falte (31) entgegengesetzte Öffnung (41),
f) Verschließen der Öffnung (41) durch eine zweite Verbindungsnaht (43),
**dadurch gekennzeichnet,**
**dass** das Falten der Materialbahn (15) im Schnitt c) derart erfolgt, dass der Streifen (14) sich an der Innenseite des Abschnitts (47) befindet, und
**dass** der als Träger für den Streifen (14) dienende Abschnitt (47) eine Klappe (46) des Beutels (40) bildet.

2. Verfahren zur Herstellung eines geschlossenen Verpackungsbeutels nach Anspruch 1, wobei der Schritt a) die folgenden Unterschritte aufweist:
a1) Entlangbewegen einer Materialbahn (10) an einer Schneidvorrichtung (13) zum Abtrennen des Streifens (14) von der Materialbahn,
a2) Versetzen des Streifens (14) relativ zu der Materialbahn (10) seitlich zur Bewegungsrichtung zur Bildung der Überlappungszone zwischen dem Streifen und der Materialbahn.

3. Verfahren zur Herstellung eines geschlossenen Verpackungsbeutels nach den Ansprüchen 1 oder 2, wobei die erste Verbindungsnaht (30) nahe des inneren Randes (27) des Streifens (14) gebildet wird.

4. Verfahren zur Herstellung eines geschlossenen Verpackungsbeutels nach einem der Ansprüche 1 bis 3, wobei als Materialbahn eine Aluminiumfolie verwendet wird, die mindestens einseitig eine durchsichtige Kunststoffbeschichtung aufweist.

5. Verfahren zur Herstellung eines geschlossenen Verpackungsbeutels nach einem der Ansprüche 1 bis 4, wobei die Verbindungsnähte (30; 43) und die Quernähte (35) als aufreißbare Nähte erzeugt werden.

6. Verfahren zur Herstellung eines geschlossenen Verpackungsbeutels nach Anspruch 2, wobei die Verbindungsnähte (30; 43) und/oder die Quernähte (35) Siegelnähte sind.

7. Verfahren zur Herstellung eines geschlossenen Verpackungsbeutels nach einem der Ansprüche 1 bis 5, wobei die Verbindungsnähte (30; 43) und/oder die Quernähte (35) Klebenähte sind.

8. Verfahren zur Herstellung eines geschlossenen Verpackungsbeutels nach einem der Ansprüche 1 bis 7, wobei durch die erste Verbindungsnaht (30) eine Lasche (44) begrenzt wird, die am entgegengesetzten Rand (26) frei liegt.

9. Verfahren zur Herstellung eines geschlossenen Verpackungsbeutels nach einem der Ansprüche 1 bis 8, wobei die erste Verbindungsnaht (30) etwa auf halber Höhe des Beutels verläuft.

10. Verfahren zur Herstellung eines geschlossenen Verpackungsbeutels nach Anspruch 9, wobei die Lasche (44) etwa die Hälfte der Höhe bedeckt, auf der die Verbindungsnaht (30) verläuft.
